# EUROPEAN PATENT SPECIFICATION

(11) **EP 2 262 910 B1**
(45) Date of publication and mention of the grant of the patent: **06.06.2012**
(21) Application number: 09727970.7
(22) Date of filing: 20.03.2009
(51) Int. Cl.: C12Q 1/68

(54) **DETECTION AND ENUMERATION OF MICROORGANISMS**
NACHWEIS UND AUFZÄHLUNG VON MIKROORGANISMEN
DÉTECTION ET DÉNOMBREMENT DE MICROORGANISMES

(30) Priority: 04.04.2008 GB 0806136; 20.01.2009 GB 0900848
(43) Date of publication of application: 22.12.2010
(73) Proprietor: BASF SE, 67056 Ludwigshafen (DE); Centre National de la Recherche Scientifique (CNRS), 75794 Paris Cedex 16 (FR)
(72) Inventor: FOVET, Yannick, 67063 Ludwigshafen (DE); DUKAN, Sam, F-13004 Marseille (FR); DUCRET, Adrien, F-13008 Marseille (FR)
(74) Representative: Peatfield, Jeremy William
(86) International application number: PCT/EP2009/053299
(87) International publication number: WO 2009/121727

(56) References cited:
- EP-A- 1 852 512
- BARCINA ISABEL ET AL: "Direct viable count of Gram-positive and Gram-negative bacteria using ciprofloxacin as inhibitor of cellular division" JOURNAL OF MICROBIOLOGICAL METHODS, vol. 22, no. 2, 1995, pages 139-150, XP002540782 ISSN: 0167-7012
- RAHMAN I ET AL: "Potential virulence of viable but nonculturable Shigella dysenteriae type 1." APPLIED AND ENVIRONMENTAL MICROBIOLOGY JAN 1996, vol. 62, no. 1, January 1996 (1996-01), pages 115-120, XP002540783 ISSN: 0099-2240
- JOUX FABIEN ET AL: "Ecological implications of an improved direct viable count method for aquatic bacteria" APPLIED AND ENVIRONMENTAL MICROBIOLOGY, vol. 63, no. 9, 1997, pages 3643-3647, XP002540784 ISSN: 0099-2240
- PINE L ET AL: "Role of keto acids and reduced-oxygen-scavenging enzymes in the growth of Legionella species." JOURNAL OF CLINICAL MICROBIOLOGY JAN 1986, vol. 23, no. 1, January 1986 (1986-01), pages 33-42, XP002540817 ISSN: 0095-1137
- WEISS E ET AL: "Substrate utilization by Legionella cells after cryopreservation in phosphate buffer." APPLIED AND ENVIRONMENTAL MICROBIOLOGY AUG 1984, vol. 48, no. 2, August 1984 (1984-08), pages 380-385, XP002540818 ISSN: 0099-2240
- TESH M J ET AL: "INTERMEDIARY METABOLISM IN LEGIONELLA-PNEUMOPHILA UTILIZATION OF AMINO-ACIDS AND OTHER COMPOUNDS AS ENERGY SOURCES" JOURNAL OF BACTERIOLOGY, vol. 154, no. 3, 1983, pages 1104-1109, XP002540819 ISSN: 0021-9193
- BESNARD V ET AL: "Development of a direct viable count procedure for the investigation of VBNC state in Listeria monocytogenes." LETTERS IN APPLIED MICROBIOLOGY JUL 2000, vol. 31, no. 1, July 2000 (2000-07), pages 77-81, XP002540785 ISSN: 0266-8254
- BAUDART JULIA ET AL: "Rapid and sensitive enumeration of viable diluted cells of members of the family enterobacteriaceae in freshwater and drinking water." APPLIED AND ENVIRONMENTAL MICROBIOLOGY OCT 2002, vol. 68, no. 10, October 2002 (2002-10), pages 5057-5063, XP002540786 ISSN: 0099-2240

## Description

The present invention concerns a method for detecting and enumerating viable microorganisms of the species *Legionella pneumophila* in a sample. The invention also includes a kit suitable for use in such a method. This method and kit enable viable microorganisms to be quantified more rapidly.

*Legionella* bacteria are ubiquitous in wet or moist environments such as soil and non-marine aquatic habitats. They can also be found in warm and cold water installations, cooling towers of air conditioning systems and water humidifiers.

*Legionella,* especially *Legionella pneumophila,* are pathogens that can cause an acute bacterial pneumonia, generally known as "legionnaires disease", which is often lethal for infected individuals.

Traditionally detection and enumeration of *Legionella pneumophila* are achieved by cell culturing. This method may be achieved by measuring culturable bacteria using plate count or measuring micro-colonies employing a filter membrane method. These techniques evaluate viable bacteria by their ability to form a colony or micro-colony. Unfortunately, such methods usually require between 3 and 10 days in order to allow the colonies or micro-colonies to form. Where water installations are still in operation there is an unacceptable risk of human infection during this time.

Other methods for detecting total *Legionella* microorganisms include PCR (Polymerase Chain Reaction) techniques. PCR employs DNA polymerase to amplify a piece of DNA by *in vitro* enzymatic replication. During the progression of the technique the DNA generated is used as a template for replication which brings about a chain reaction in which the DNA template is exponentially amplified. PCR enables a single or few copies of a piece of DNA to be amplified by generating millions or more copies of the DNA piece. Typically such a method is described by Diederen et al., J Med Microbiol. 2007 Jan; 56 (Pt 1):94-101.

However a drawback of PCR is that the samples tend to contain polymerisation reaction inhibitors and therefore do not consistently provide quantitative results. Furthermore, the technique relies upon a prior DNA purification step which can result in loss of DNA with the consequential underestimation of the *Legionella* present. To some extent these disadvantages are overcome by real-time PCR which is quantitative. However, the technique cannot distinguish between viable cells and non-viable cells.

Another technique is fluorescent *in situ* hybridisation (FISH) in which an oligonucleotidic probe labelled by a fluorescent substance penetrates into the bacteria cells. Where the ribosomal nucleic acids (rRNA) have the correct sequence to the probe known as the target, the probe will attach itself to its target and will not be removed by any subsequent washing step. The bacteria in which the probe is fixed will then emit a fluorescent signal. This fluorescent signal may then be quantified by techniques such as flow cytometry, solid phase cytometry, or epifluorescent microscopy. A typical FISH technique is described by Dutil S et al J Appl Microbiol. 2006 May;100(5):955-63. However, using the FISH technique alone the total number of viable *Legionella pneumophila* could be detected but unfortunately the method could not exclusively identify only those *Legionella pneumophila* bacteria able to divide and by consequence make a colony.

A further method for enumerating viable *Legionella pneumophila* involves ChemChrome V6 and is described by Delgado-Viscogliosi et al Appl Environ Microbiol. 2005 Jul;71(7):4086-96. This method allows the quantification of *Legionella pneumophila* as well as discrimination between viable and non-viable bacteria. It combines specific detection of *Legionella* cells using antibodies and a bacterial viability marker (ChemChrome V6) and employing epifluorescent microscopy for the enumeration. However, although this technique distinguishes between viable and non-viable cells it is not able to separately identify those colony-forming bacteria.

To date the only methods which permit detection of *Legionella pneumophila* bacteria able to divide has been based on the micro colony method (Scan-VIT). However this method requires around 72 hours.

Arana et al. " Detection and enumeration of viable but non-culturable transconjugants of Escherichia coli during the survival of recipient cells in river water" pp 340-346, J. App. Microbiol. Vol 83, 1997 describes the use of direct viability count (DVC) using specific markers.

Piqueres et al. "A combination of direct viable count and fluorescent in situ hybridisation for estimating Helicobacter pylori cell viability" pp 345-349 Res. Macrobiol. Vol 157, 2006 Jjemba et al. "In situ enumeration and probing of pyrene degrading soil bacteria" pp 287-298 FEMS Microbiol. Ecol. Vol. 55, 2006 both refer to DVC-FISH but for microorganisms unrelated to *Legionella pneumophila.*

EP-A-1852512 describes a method for identification and enumeration of several pathogenic microorganisms including *Legionella pneumophila.* The method incorporates the direct viability count (DVC) and the fluorescence in situ hybridisation (FISH) but also employs a helper probe in order to amplify the signal. Helper probes are unlablled oligonucleotides that bind to regions adjacent to that targeted by the specific labelled probe. This enhances in situ accessibility and hence the probe conferred signal.

The method described in EP-A-1852512 is said to employ a DNA gyrase inhibitor such as nalidixic acid, which stops cell division, increases the intracellular rRNA content and the cellular length of sensitive cells. However, in practice nalidixic acid is not a reliably effective DNA gyrase inhibitor for *Legionella pneumophila.* Furthermore, this document does not mention problems of inaccurate enumeration that can occur due to the presence of naturally fluorescent microorganisms.

It would be desirable to provide a method for reliably quantifying viable *Legionella pneumophila* capable of forming colonies in a sample more rapidly than known techniques. In addition, it would be preferable to achieve this more accurately.

According to the present invention we provide a method for detecting and enumerating viable microorganisms in a sample suspected of containing said microorganisms comprising:
(1) contacting said sample with a cell nutritive resource and a cellular proliferation inhibitor, in which the cell nutritive resource contains a growth supplement which comprises an antioxidant, preferably pyruvic acid (or salt thereof),
(2) contacting said sample with at least one fluorescence labelled oligonucleotidic probe able to specifically hybridise at least one portion of ribosomal nucleic acids of said microorganisms, in which said sample is contacted with at least a first probe and a second probe in which the first probe is able to specifically hybridise at least one portion of ribosomal nucleic acids of said microorganisms and the second probe is able to specifically hybridise at least one different portion of ribosomal nucleic acids of the microorganisms,
(3) detecting and quantifying the fluorescent signal,
in which the microorganisms are of the species *Legionella pneumophila* and in which the cellular proliferation inhibitor is selected from the group consisting of ciprofloxacin and cephalexin.

Generally each of the steps are carried out sequentially in which step (2) is performed on the sample so treated in step (1) and then step (3) is conducted following step (2).

Step 1 of the inventive method is known as Direct Viable Count (DVC) which is based on incubating a bacteria in the presence of an antibiotic or DNA gyrase inhibitor which blocks the cellular division without deteriorating the cellular metabolism. Therefore the living bacteria will tend to elongate but not divide and so can be distinguished from the dead bacteria which do not change in size. It is therefore possible to identify living bacteria by microscopy. Prior to carrying out the DVC step it may be desirable to concentrate the sample and to pre-treat it for instance by acid and/or heat treatment according to the standard method T 90-431 (ISSN 0335-3931), edited and distributed by the French Association of Normalization (AFNOR) 11, Avenue Francis de Pressensé-93571 St Denis La Plaine Cedex, France.

We have found unexpectedly that ciprofloxacin and cephalexin are very effective DNA gyrase inhibitors. Both of ciprofloxacin and cephalexin allow the *Legionella pneumophila* cells to elongate in preparation for cell division but actually block these elongated cells from dividing. By contrast nalidixic acid does not provide sufficient elongation of the *Legionella pneumophila* cells to be effective for the present method. Advantageously we find that the method of the present invention enables *Legionella pneumophila* to be identified and quantified reliably and within a timescale normally of less than 24 hours. This provides significant improvements in water sanitary control.

The ciprofloxacin or cephalexin may be used in any effective amount. Typically this would be in concentrations of up to 20 mg/L or higher within the medium contained in the cell nutritive resource. Preferably the concentrations are between 1 and 10 mg/L. Preferably the cellular proliferation inhibitor is ciprofloxacin. We have found the greatest cellular elongation can be achieved when the concentration of ciprofloxacin is between 2 and 6 mg/L after a duration of 12 hours, especially between 3 and 5 mg/L.

The cell nutritive resource should contain any suitable growth medium composition applicable to the DVC method and suitable for *Legionella pneumophila.* A suitable medium composition may comprise a medium, selective supplement, cellular proliferation inhibitor (ciprofloxacin or cephalexin), and growth supplement.

The medium provides the minimum nutrition required to allow growth of *Legionella pneumophila.* It may be any suitable medium as described in the literature, for instance according to the standard method prescription T 90-431 (as given above) without agar and charcoal.

The selective supplement is often required in order to limit the development of interfering microorganisms. The choice of antibiotic may be any known supplement suitable for the DVC method, for instance has described in standard method T 90-431 (as given above). Nevertheless the concentration of each antibiotic in general should be adapted for the particular liquid medium. However, it would not necessarily be detrimental in cases where these other microorganisms are not completely eliminated since step 2 will usually be sufficiently specific to overcome the effect of interfering bacteria.

Although the growth supplement is not essential to allow growth of the *Legionella pneumophila* bacteria, it can optimise its growth. *Legionella pneumophila* is characterised by doubling in 120 min (under optimum conditions). However, we have found that in some cases the viable *Legionella pneumophila* bacteria which are able to form colonies may not necessarily form colonies immediately and instead undergo a delay or lag phase before growth commences. This lag phase can be between 8 to more than 20 hours in the function of initial physiological state.

In the present invention, the lag phase for *Legionella pneumophila* can be reduced by including as the growth supplement and antioxidant reagent into the cell nutritive resource. The antioxidant reagent may act directly upon reactive oxygen species (ROS) or by other means such as causing an effect on the metabolism of the microorganism, directly or indirectly, which brings about a reduction in ROS. Suitable antioxidant reagents include catalase, ascorbic acid, sodium metabisulphite, dimethyl sulfoxide, TDPA (3,3'-thiodipropionic acid) and pyruvate etc. Preferably antioxidant reagent is pyruvate. Preferred doses of antioxidant reagent, especially for pyruvate, are between 0.5 and 1.5 g/L, especially around 1 g/L.

The cell nutritive resource may include at least one compound that indirectly inhibits the formation of and/or degrades the Reactive Oxygen Species (ROS), said compound may bring about reduced levels of ROS by interfering with the metabolism of the microorganism. Typically such compounds will include amino acids or their salts. A particularly preferred compound is glutamic acid or glutamate salt.

In a still further preferred form of the invention the cell nutritive resource would include glutamic acid or glutamate salt, especially the sodium salt. In general the amount of glutamic acid or glutamate will be between 0.01 and 5% by weight calculated as the sodium salt.

It is particularly preferred that the cell nutritive resource includes both pyruvic acid or pyruvate (especially the sodium salt) together with glutamic acid or glutamate (especially as the sodium salt). This combination of pyruvic acid or pyruvate with glutamic acid or glutamate seems to induce a synergistic effect in that it allows a higher estimation (and therefore more accurate estimation) of culturable *Legionella* than either compound respectively used alone. Furthermore we have found that this combination brings about a further reduction of lag phase during development of the *Legionella pneumophila,* in particular in a liquid medium. Such a reduction of lag phase in liquid medium results in a reduction of the time required to obtain a visible colony on agar plate.

Desirably the amount of pyruvate and glutamate will be as stated previously. It is particularly preferred that the ratio of glutamate to pyruvate will be in the range between 1:1 and 50:1, especially between 5:1 and 20:1 and more especially between 7:1 and 15:1.

Glutamate is not known to be an antioxidant. However, it s appear that indirectly glutamate could reduce the endogenous production of ROS naturally formed during growth or their consequences on macromolecules (oxidation).

Without being limited to theory, it thought that the glutamic acid changes the metabolism of *Legionella* to increase the effect of pyruvate and that this interference with the metabolism of *Legionella* indirectly inhibits the formation of and/or degrades intracellular ROS.

The method of the present invention can enable a shorter incubation period to be employed, desirably no more than about 24 hours. Preferably this can be reduced to 12 hours, especially when there is a low occurrence of bacterial interference and/or high occurrence of *Legionella pneumophila* bacteria.

The sample may be collected from any suitable location. This may for instance be a sample of water from recirculating water in a cooling system. However, desirably the sample may be obtained from water in the form of an aerosol. Typically the aerosol may be located in a cooling tower or air conditionner. Desirably the water condensed from the aerosol before testing according to the method of the present invention.

The mode of incubation can be as defined in literature describing the DVC procedure. Such a method can include sample filtration and then filter incubation on pads soaked with the medium. Preferably according to the present invention the sample, or concentrated sample, is directly incubated with the medium and then filtrated. In this way we can reduce the risk of losing bacteria during the incubation process and provide better conditions during incubation, for instance oxygen transfer or agitation of the sample.

Step 2 of the inventive method can employ a standard FISH protocol.

In the first part of the FISH procedure the cells may be fixed by treating the external membrane or envelope of the cell to make it permeable to the oligonucleotidic probes. Generally this will be achieved by using fixing solutions that are aqueous solutions of alcohols or aldehydes which are miscible with water at 25°C. Suitable alcohols or aldehydes include formaldehyde, paraformaldehyde, ethanol and/or methanol. Typically solutions of formaldehyde or paraformaldehyde can be up to 10% by weight and preferably between 1 and 5%. Usually the alcohols will be at least 50% by weight and generally between 60 and 90% by weight. Preferably this treatment can be achieved by sequential treatment with one or more of these solutions. Preferably the treatment may comprise a solution of between 1 and 5% formaldehyde or paraformaldehyde followed by two or three solutions of ethanol or methanol of increasing strength between 50% and 90%.

The FISH procedure then employs a hybridisation procedure by employing a hybridisation buffer containing at least one fluorescence labelled oligonucleotidic probe comprising an oligonucleotide with attached fluorescent marker in which the oligonucleotide is capable of targeting a specific sequence within the cell. The oligonucleotidic probes penetrate the external membrane of the cells and bind to the target sequence corresponding to the oligonucleotide. Binding will be understood to mean the formation of hydrogen bonds between complimentary nucleic acid pieces. In the FISH technique the oligonucleotidic probes are complimentary and capable of binding to a certain region of the ribosomal target sequence within the microorganism. Typically the probes comprise between 15 and 30 bases in length as single-stranded deoxyribonucleic acid pieces and are directed to a specific target region specific to the microorganism.

The oligonucleotidic probe should desirably be a specific ribosomal ARN 16S probe of *Legionella pneumophila.* Any oligonucleotidic probes which specifically target microorganisms of the species *Legionella pneumophila* may be employed. Preferably the probe will be selected from the group consisting of PNE 1 probe described by Grimm et al 1998 with a 5' 3' base sequence of ATC TGA CCG TCC CAG GTT, LEGPNE 1 probe (SEQ ID No 22) described by Grimm et al 1998 and Declerck et al 2003 having a 5' 3' base sequence of ATCTG ACCGT CCCAG GTT, and LP2 probe (SEQ ID No 23) described by Yamamoto et al 1993 having a 5' 3' base sequence of AGCTT TCATC CAAAG ATA.

It may also be desirable to alternatively employ probes with sequences having at least 70%, preferably at least 80% and more preferably at least 90% identity with any of the three probes PNE 1 probe, LEGPNE 1 probe, or LP2 probe.

In order to overcome the risk of falsely identified microorganisms that are naturally fluorescent, the invention employs two nucleotidic probes. One probe targets all microorganisms from the *Legionella* genus and the second probe is designed to hybridise specifically microorganisms of *Legionella pneumophila* species. Each probe is labelled with different fluorescent dyes. The microorganisms that are naturally fluorescent will be fluorescent in only a specific wavelength or narrow band of wavelengths and consequently using the two probes with different dyes that fluoresce at different wavelengths allows naturally fluorescent microorganisms that are not specifically *Legionella pneumophila* to be eliminated.

In the invention, the first probe will hybridise microorganisms belonging to the *Legionella* genus, which includes but is not limited to *Legionella longbeachae, Legionella jordanis, Legionella anisa, Legionella pneumophila.* This first probe can comprise a nucleotide that can bind with a target ribosomal sequence from any of the bacteria within the *Legionella* genus. Typically the first probe can be any of the probes selected from the group consisting of the LEG705 probe (SEQ ID n°7) described in Manz *et al.* (Manz *et al.* 1995) with a 5' 3' base sequence of CTGGT GTTCC TTCCG ATC, the LEG226 probe (SEQ ID n°8) described in Manz *et al.* (Manz *et al.* 1995), with a 5' 3' base sequence of TCGGA CGCAG GCTAA TCT, the Legall11 probe (SEQ ID n°9) described in Leskela *et al.* (Leskela *et al.* 2005) with a 5' 3' base sequence of CCTCC TCCCC ACTGA AAGT, the Legall22 probe (SEQ ID n°10) described in Leskela *et al.* (Leskela *et al.* 2005) with a 5' 3' base sequence of CACTG TATGT CAAGG GTAGG, the Leg120v probe (SEQ ID n°11) described in Buchbinder *et al.* (Bushbinder *et al.* 2002) with a 5' 3' base sequence of AAGGC ATATT CCTAC GCG.

It may also be desirable to alternatively employ probes with sequences having at least 70%, preferably at least 80% and more preferably at least 90% identity with any of the three probes LEG705 probe, LEG226 probe, Legall11 probe, Legall22 probe, and Leg120v probe.

The second probe can hybridise only the specific species of *Legionella pneumophila* and can be any of the aforementioned nucleotidic probes with this characteristic e.g. any of the three probes PNE 1 probe, LEGPNE 1 probe, or LP2 probe.

Any of the fluorescent dyes known to be compatible with the appropriate emission/excitation spectra of FITC (for instance Syto9, Alexa 488 etc) or Cy3 (for instance Rhodamine, Alexa 583 etc) can be used.

Step 3 involves quantifying relevant bacteria using a microscope. This can be achieved manually or automatically, for instance using an epifluorescent microscope. Preferably the detection and counting of bacteria labelled by *in situ* hybridization and fixed on a filter need the use of microscope equipped with an epifluorescence system.

Suitable detection devices include ChemScan RDI and ScanVIT- Legionella TM (Vernicon AG, Munich, Germany). It possible to use chemscan (solid cytometry developed by AESChemunex) to detect and count labelled bacteria. However, this system can be use only 1 set of emission/excitation mirror (488 nm) and thus limit our protocol to use only one labelled probe.

ScanVIT- Legionella TM is preferred especially according to the aforementioned preferred aspect of the invention employing at least two probes, since this technique permits the use of two different fluorescent signals in order to eliminate naturally fluorescent microorganisms that are not *Legionella pneumophila.*

The method according to present invention facilitates the accurate and rapid quantitative determination for the existence of *Legionella pneumophila.* The method is suitable for detecting *Legionella pneumophila* in samples derived from any of the group selected from industrial cooling waters, drinking waters, and natural waters.

The present invention also incorporates a kit for more rapidly detecting and enumerating viable microorganisms of the species *Legionella pneumophila* in a sample suspected of containing said microorganisms comprising:
(1) a cell nutritive resource, which contains a growth supplement which comprises an antioxidant, preferably pyruvic acid (or salt thereof),
(2) a cellular proliferation inhibitor,
(3) fluorescence labelled oligonucleotidic probes able to specifically hybridise at least one portion of ribosomal nucleic acids of said microorganisms, comprising a first probe and a second probe in which the first probe able to specifically hybridise at least one portion of ribosomal nucleic acids of said microorganisms and the second probe able to specifically hybridise at least one different portion of ribosomal nucleic acids of the microorganisms

### (4) a means for detecting and quantifying the fluorescent signal, in which the cellular proliferation inhibitor is selected from the group consisting of ciprofloxacin and cephalexin.

In a preferred form a kit may comprise the following solutions: a medium composition (as defined above) which can be dehydrated for long storage; a solution for fixing the external membrane of bacteria, for instance formaldehyde; a hybridisation solution, for instance as described above, which should be made up shortly before use; a wash solution, for instance as described above, which should be made up shortly before use; nucleic acid fluorescence dye, for instance DAPI which could be dehydrated for long storage; an anti fading mounting reagent. Preferably the kit may comprise filters. More preferably the kit may additionally comprise a physiological buffer; ethanol solutions of strength varying between 50 and 90%; and sterile water. The kit may also contain Eppendorf, for instance 2 ml.

Kit may also contain any of the embodiments described in regard to the first aspect of the invention.

The kit is suitable for use with the method of the present invention and enables rapidly and reliable enumeration of *Legionella pneumophila.*

The following examples illustrate the invention.

### Example 1

*A Legionella pneumophila* suspension at final concentration of 10⁶ bacteria/ml is dispatched in 2 suspensions of same volume. Only the first suspension (S1) is fixed with 3.7 % (v/vl) formaldehyde at ambient temperature (20 to 22 °C) for 30 min. The both suspension are then washed three times by centrifugation (6,000 x g, 5 min at 20°C), in PBS pH 7.4.

The both suspension are finally mixed in accord with table 1.

**Table 1**

| % viable Cell | S1 [ml] | S2 [ml] |
|---|---|---|
| 100 | 0 | 2 |
| 50 | 1 | 1 |
| 10 | 1.8 | 0.2 |
| 1 | 1.98 | 0.02 |
| 0 | 2 | 0 |

Each mix is treated according to the experimental protocol below.

### Results

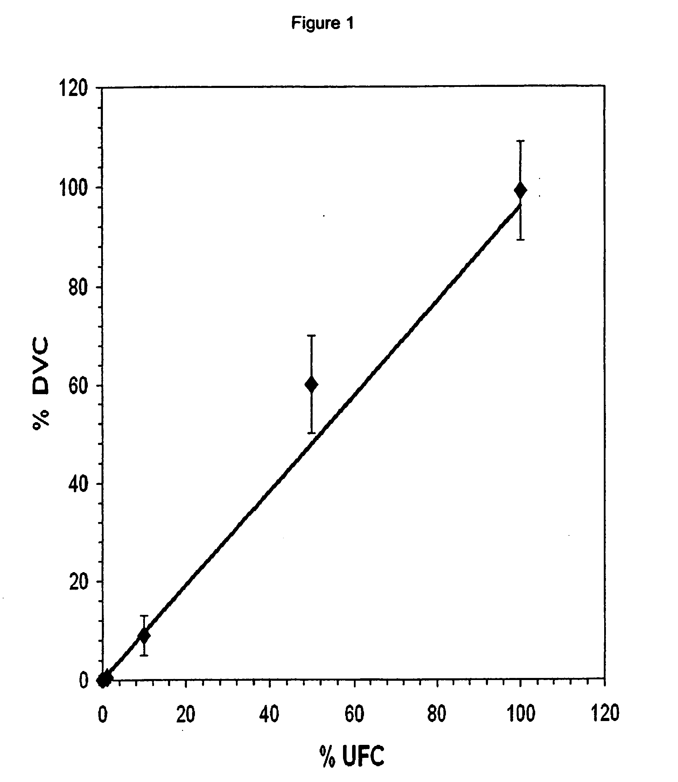

The results are represented in figure 1. Figure 1 shows the correlation between the standard method AFNOR (8 days) and the method of the present invention requiring less than 24 hours. The results indicate a good correlation between the two methods in terms of accuracy of identification of *Legionella pneumophila.*

### Experimental protocol

Samples (V₁) were filtered through 25-mm-diameter, 0.2-µm-pore-size white polycarbonate membrane (Millipore, GTTP02500). Filters were rinsed two times with 10 ml of Solution A and were disposed in a sterile tube (Eppendorf 2ml) containing 1 ml of solution A. Tubes were shaken 1 min at 30Hz (4°C). After filters removing, each suspension was dispatched like follow:
1. 500 µl of suspension is disposed aseptically in a sterile tube containing 500 µl of solution B. Tubes were then incubated 24 hours at 37°C or 45°C with agitation. After incubation, suspensions were filtered through 25-mm-diameter, 0.2-µm-pore-size white polycarbonate membrane (Millipore, GTTP02500) and were fixed with solution C at ambient temperature (20 to 22°C) for 30 min. Filters were rinsed two times with phosphate buffered saline (pH 7.4) and air dried
2. 500 µl of suspensions were directly filtered through 25-mm-diameter, 0.2-µm-pore-size white polycarbonate membrane (Millipore, GTTP02500) and were fixed with solution C at ambient temperature (20 to 22°C) for 30 min. Filters were rinsed two times with phosphate buffered saline (pH 7.4) and air dried

Filters were dehydrated by sequential washes in Solution D, Solution E and Solution F (2 min each) and then dried by incubation 30 min at 37°C. Filters were disposed on slide and 50 µl of Solution G was applied to the filter. A cover slip was disposed on filter to avoid dehydration. Hybridization was performed for 2 hours at 46 ± 1°C in a moisture chamber. Then the filters were washed three times 5 ml of solution H preheated at 46°C and then rinsed two times with sterile water. Filters were incubated 15 min with 1 ml of Solution I and rinse two times with sterile water. After air dried, filters were finally mounted on a slide with 20 µl of solution J. Hybridized cells were visualized by epifluorescence microscopy with an x100 immersion objective lens and 2 emission/excitation filters: a 510 to 550 nm excitation filter and a 590 nm barrier filter.

### Solutions

**Table 2**

| Solution | Details | General Description |
|---|---|---|
| A | Posphate Buffer Saline pH 7.4 (PBS) | Physiological buffer |
| B | Medium | See Table 3 |
| C | Formaldehyde [3.7%] (Sigma, F-1635) | |
| D | Ethanol 50% | |
| E | Ethanol 80% | |
| F | Ethanol 90% | |
| G | Hybridization Solution | See Table 4 |
| H | Wash Solution | See Table 5 |
| I | DAPI [0,5µg/ml] (Sigma, D-95421) | Nucleic Acid Fluorescent Dye |
| J | Citifluor (Ted Pella, INC., 19476-A) | Anti-fading Mounting Reagent |

### Medium 2X

**Table 3**

| | Compounds | Concentration | CAS |
|---|---|---|---|
| | Yeast Extract | 20 g/L | |
| | Ferric Pyrophosphate | 0.5 g/L | |
| | L-Cysteine | 0.8 g/L | |
| Medium | (chlorydrate) | | |
| | α-ketoglutarate | 2 g/L | |
| | Buffer | | |
| | ACES/Potassium | 2 g/L | |
| | Hydroxide | | |
| | Glycine | 6 mg/L | |
| Selective | Vancomycine | 2 µg/L | |
| Supplement | Polymyxine | 160 Ul/L | |
| | Cycloheximide | 160 µg/L | |
| Cell Division Blocking Antibiotic | Ciprofloxacin | 8 mg/L | 85721-33-1 |
| Growth Supplement | Pyruvate | 2 g/L | |

All reactive were sterilized by filtration trough 0.2-µm-pore-size nitrocellulose membrane (Millipore, SLGS025 OS). For details of each set of reagent see below.

### Hybridization solution

**Table 4**

| Compounds | Concentration |
|---|---|
| formamide (Sigma, F-9037) | 20% |
| NaCl (Sigma, S-9625) | 0.9 M |
| SDS (Sigma, L-4522) | 0.1 % |
| Tris-HCl pH 7.2 (Sigma, T-2538) | 20 mM |

All reactive were sterilized by filtration trough 0.2-µm-pore-size nitrocellulose membrane (Millipore, SLGS025 OS).

Probe used for FISH detection are: LEG705 (Eurogentec: 5'-CTGGTGTTCCTTCCGATC-3'), specific of *Legionellaceae* and labelled with FITC (Fluorescéine isothiocyanate) and PNE1 (Eurogentec: 5'-CTGGTGTTCCTTCCGATC-3'), specific of *Legionella pneumophila* genus and labelled with Cy3.

Probes were added to hybridization solution at final concentration of 1 ng/µl.

### Wash solution

**Table 5**

| Compounds | Concentration |
|---|---|
| NaCl (Sigma, S-9625) | 215 mM |
| SDS (Sigma, L-4522) | 0.1 % |
| Tris-HCl pH 7.2 (Sigma, T-2538) | 20 mM |

All reactive were sterilized by filtration trough 0.2-µm-pore-size nitrocellulose membrane (Millipore, SLGS025 OS).

### Example 2

The experimental protocol of Example 1 is applied to a sample employing leg705 first probe labelled with a green fluorescent dye and PNE1 second probe labelled with a red fluorescent dye.

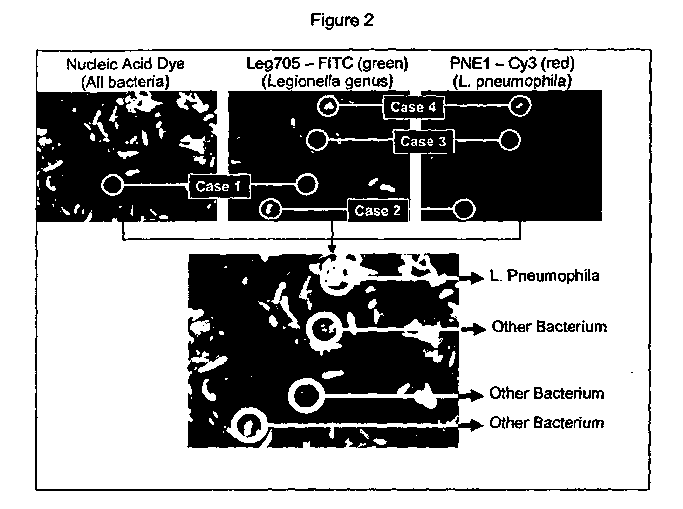

Figure 2 shows four cases which allow *Legionella pneumophila* to be distinguished from naturally fluorescent bacteria. Referring to Figure 2:
Case 1-Neither Green, neither red fluorescent bacterium - bacterium doesn't belong to *Legionella* genus and to *Legionella pneumophila* species;
Case 2-Only Green fluorescent bacterium - the bacterium belongs to *Legionella* genus OR is naturally fluorescent;
Case 3-Only Red bacterium - Despite red fluorescence, bacterium does not belong to *Legionella pneumophila* species because it is not green fluorescent and thus doesn't belong to *Legionella* genus. This bacterium is naturally red fluorescent; and
Case 4-Green AND Red fluorescent bacterium - the bacterium belongs to *Legionella* genus AND *Legionella pneumophila* species.

Using this method we can accurately detect *Legionella pneumophila* by limiting the detection of naturally fluorescent bacteria which would otherwise falsely indicate this bacteria.

The PNE1 probe is first described in Grimm *et al.* (Grimm *et al.,* 1998). Specific sequence is : 5'-ATC TGA CCG TCC CAG GTT-3'

The Leg705 probe is first described in Manz *et al.* (Manz *et al.,* 1995). Specific sequence is : 5'-CTGGTGTTCCTTCCGATC-3'

Three dyes were used in this test are used as follows:
The first dye is used to stain nucleic acid of all micro organisms. DAPI is used to stain bacteria in blue under UV excitation. This dye is not coupled with an oligonucleotidic probe.

The second two dyes are coupled with oligonucleotidic probe to allow specific detection. These two dyes must be characterized by two different spectra of excitation/emission. In the test FITC and Cy3 are used, which are the most commonly dyes used to FISH detection, but many dyes with respectively the same spectra are available.

**Table 6**

| Dyes used | λ Excitation [nm] | λ Emission [nm] | Dyes Available |
|---|---|---|---|
| DAPI | 350 | 461 | |
| FITC | 494 | 521 | Alexa 488, Hylite 488, Dylight 488, |
| Cy3 | 550 | 570 | Rhodamine, Alexa 555, Hylite 555, ... |

## Claims

1. A method for detecting and enumerating viable microorganisms in a sample suspected of containing said microorganisms comprising:
(1) contacting said sample with a cell nutritive resource and a cellular proliferation inhibitor, in which the cell nutritive resource contains a growth supplement which comprises an antioxidant, preferably pyruvic acid (or salt thereof),
(2) contacting said sample with at least one fluorescence labelled oligonucleotidic probe able to specifically hybridise at least one portion of ribosomal nucleic acids of said microorganisms, in which said sample is contacted with at least a first probe and a second probe in which the first probe is able to specifically hybridise at least one portion of ribosomal nucleic acids of said microorganisms and the second probe is able to specifically hybridise at least one different portion of ribosomal nucleic acids of the microorganisms,
(3) detecting and quantifying the fluorescent signal, in which the microorganisms are of the species *Legionella pneumophila* and in which the cellular proliferation inhibitor is selected from the group consisting of ciprofloxacin and cephalexin.

2. A method according to claim 1 in which the cell nutritive resource of step 1) contains glutamic acid (or salt thereof).

3. A method according to claim 1 or claim 2 in which the cell nutritive resource of step 1) contains glutamic acid (or salt thereof) and pyruvic acid (or salt thereof).

4. A method according to any preceding claim in which the oligonucleotidic probe is selected from the group consisting of probe with a 5' 3' base sequence of ATC TGA CCG TCC CAG GTT; probe having a 5' 3' base sequence of ATCTG ACCGT CCCAG GTT; and probe having a 5' 3' base sequence of AGCTT TCATC CAAAG ATA.

5. A method according to any preceding claim in which in step (2) said sample is contacted with at least a first probe and a second probe in which the first probe is able to specifically hybridise at least one portion of ribosomal nucleic acids all belonging to *Legionella* genus and in which the second probe is able to specifically hybridise at least one portion of the ribosomal nucleic acids all belonging to *Legionella pneumophila* species.

6. A method according to claim 5 in which the first probe is selected from the group consisting of probe with a 5' 3' base sequence of CTGGT GTTCC TTCCG ATC; probe with a 5' 3' base sequence of TCGGA CGCAG GCTAA TCT; probe with a 5' 3' base sequence of CCTCC TCCCC ACTGA AAGT; probe with a 5' 3' base sequence of CACTG TATGT CAAGG GTAGG; and probe with a 5' 3' base sequence of AAGGC ATATT CCTAC GCG.

7. A method according to any preceding claim in which step (3) is performed automatically using an epifluorescent microscope.

8. A method according to any preceding claim in which the sample is derived from any of the group selected from industrial cooling waters, drinking waters, and natural waters.

9. Kit for more rapidly detecting and enumerating viable microorganisms of the species *Legionella pneumophila* in a sample suspected of containing said microorganisms comprising:
(1) a cell nutritive resource, which contains a growth supplement which comprises an antioxidant, preferably pyruvic acid (or salt thereof),
(2) a cellular proliferation inhibitor,
(3) fluorescence labelled oligonucleotidic probes able to specifically hybridise at least one portion of ribosomal nucleic acids of said microorganisms, comprising a first probe and a second probe in which the first probe able to specifically hybridise at least one portion of ribosomal nucleic acids of said microorganisms and the second probe able to specifically hybridise at least one different portion of ribosomal nucleic acids of the microorganisms
(4) a means for detecting and quantifying the fluorescent signal, in which the cellular proliferation inhibitor is selected from the group consisting of ciprofloxacin and cephalexin.

## Patentansprüche

1. Verfahren zum Nachweisen und Zählen von lebensfähigen Mikroorganismen in einer Probe, von der vermutet wird, die Mikroorganismen zu enthalten, welches folgendes umfasst:
(1) Inkontaktbringen der Probe mit einer Zellnährstoffquelle und einem Zellproliferationsinhibitor, wobei die Zellnährstoffquelle einen Wachstums-Zusatzstoff enthält, der ein Antioxidationsmittel, vorzugsweise Brenztraubensäure (oder Salz davon), umfasst,
(2) Inkontaktbringen der Probe mit mindestens einer fluoreszenzmarkierten Oligonukleotidsonde, die in der Lage ist, spezifisch mit mindestens einem Teil von ribosomalen Nukleinsäuren der Mikroorganismen zu Hybridisieren, wobei die Probe mit mindestens einer ersten Sonde und einer zweiten Sonde in Kontakt gebracht wird, wobei die erste Sonde zum spezifischen Hybridisieren mit mindestens einem Teil der ribosomalen Nukleinsäuren der Mikroorganismen in der Lage ist and die zweite Sonde zum spezifischen Hybridisieren mit mindestens einem anderen Teil der ribosomalen Nukleinsäuren der Mikroorganismen in der Lage ist,
(3) Nachweisen und Quantifizieren des Fluoreszenzsignals, wobei die Mikroorganismen aus der Spezies *Legionella pneumophila* sind und wobei der Zellproliferationsinhibitor aus der Gruppe, bestehend aus Ciprofloxacin und Cephalexin, ausgewählt ist.

2. Verfahren gemäß Anspruch 1, bei dem die Zellnährstoffquelle von Schritt 1) Glutaminsäure (oder Salz davon) enthält.

3. vorfahren gemäß Anspruch 1 oder Anspruch 2, bei dem die Zellnährstoffquelle von Schritt 1) Glutaminsäure
(oder Salz davon) und Brenztraubensäure (oder Salz davon) enthält.

4. Verfahren gemäß einem beliebigen vorangehenden Anspruch, bei dem die Oligonukleotidsonde aus der Gruppe, bestehend aus einer Sonde mit einer 5'-3'-Basensequenz ATC TGA CCG TCC CAG GTT; einer Sonde mit einer 5'-3'-Basensequenz ATCTG ACCGT CCCAG GTT; und einer Sonde mit einer 5'-3'-Basensequenz AGCTT TCATC CAAAG ATA, gewählt ist.

5. Verfahren gemäß einem beliebigen vorangehenden Anspruch, bei dem in Schritt (2) die Probe mit mindestens einer ersten Sonde und einer zweiten Sonde in Kontakt gebracht wird, wobei die erste Sonde zum spezifischen Hybridisieren mit mindestens einem Teil von ribosomalen Nukleinsäuren, die alle zur Gattung *Legionella* gehören, in der Lage ist, und wobei die zweite Sonde zum spezifischen Hybridisieren mit mindestens einem Teil der ribosomalen Nukleinsäuren, die alle zur Spezies *Legionella pneumophila* gehören, in der Lage ist.

6. Verfahren gemäß Anspruch 5, bei dem die erste Sonde aus der Gruppe, bestehend aus einer Sonde mit einer 5'-3'-Basensequenz CTGGT GTTCC TTCCG ATC; einer Sonde mit einer 5'-3'-Basensequenz TCGGA CGCAG GCTAA TCT; einer Sonde mit einer 5'-3'-Basensequenz CCTCC TCCCC ACTA SAGT; einer Sonde mit einer 5'-3'-Basensequenz CACTG TATGT CAAGG GTAGG; und einer Sonde mit einer 5'-3'-Basensequenz AAGGC STATT CCTAC GCG, gewählt ist.

7. Verfahren gemäß einem beliebigen vorangehenden Anspruch, bei dem der Schritt (3) automatisch unter Verwendung eines Epifluoreszenz-Mikroskops durchgeführt wird.

8. Verfahren gemäß einem beliebigen vorangehenden Anspruch, bei dem die Probe von beliebigen aus der Gruppe, gewählt aus industriellem Kühlwasser, Trinkwasser und natürlichen Gewässern, abgeleitet wird.

9. Kit zum schnelleren Nachweisen und Zählen lebensfähiger Mikroorganismen der Spezies *Legionella pneumophila* in einer Probe, von der vermutet wird, die Mikroorganismen zu enthalten, welches folgendes umfasst:
(1) eine Zellnährstoffquelle, die einen Wachstums-Zusatzstoff enthält, der ein Antioxidationsmittel, vorzugsweise Brenztraubensäure (oder Salz davon), umfasst,
(2) einen Zellproliferationsinhibitor,
(3) fluoreszenzmarkierte Oligonukleotidsonden, die in der Lage sind, spezifisch mit mindestens einem Teil von ribosomalen Nukleinsäuren der Mikroorganismen zu Hybridisieren, umfassend eine erste Sonde und eine zweite Sonde, wobei die erste Sonde zum spezifischen Hybridisieren mit mindestens einem Teil von ribosomalen Nukleinsäuren der Mikroorganismen in der Lage ist, und die zweite Sonde zum spezifischen Hybridisieren mit mindestens einem anderen Teil von ribosomalen Nukleinsäuren der Mikroorganismen in der Lage ist,
(4) eine Einrichtung zum Nachweisen und Quantifizieren des Fluoreszenzsignals, wobei der Zellproliferationsinhibitor aus der Gruppe, bestehend aus Ciprofloxacin und Cephalexin, ausgewählt ist.

## Revendications

1. Procédé pour détecter et dénombrer les micro-organismes viables dans une échantillon que l'on suspecte de convenir lesdites micro-organismes, comprenant :
(1) la mise en contact dudit échantillon avec une ressource nutritive pour les cellules et un inhibiteur de prolifération cellulaire, la ressource nutritive pour les cellules contenant un supplément de croissance, qui comprend un antioxydant, de préférence l'acide pyruvique (ou un sel de ce dernier),
(2) la mise en contact dudit échantillon avec au moins une sonde oligonucléotidique marquée par fluorescence, apte à s'hybrider d'une manière spécifique avec au moins une partie d'acides nucléiques ribosomiques desdits micro-organismes, ledit échantillon étant mis en contact avec au moins une première sonde et une deuxième sonde, la première sonde étant apte à s'hybrider d'une manière spécifique avec au moins une partie des acides nucléiques ribosomiques desdits micro-organismes, et la deuxième sonde étant apte à s'hybrider d'une manière spécifique avec au moins une partie différente des acides nucléiques ribosomiques des micro-organismes,
(3) la détection et la quantification du signal fluorescent, les micro-organismes étant de l'espèce *Legionella pneumophila*, et l'inhibiteur de prolifération cellulaire étant choisi dans le groupe consistant en la ciprofloxacine et la céphalexine.

2. Procédé selon la revendication 1, dans lequel la ressource nutritive pour les cellules de l'étape 1) contient de l'acide glutamique (ou un sel de ce dernier).

3. Procédé selon la revendication 1 ou 2, dans lequel la ressource nutritive pour les cellules de l'étape 1) contient de l'acide glutamique (ou un sel de ce dernier) et de l'acide pyruvique (ou un sel de ce dernier).

4. Procédé selon l'une quelconque des revendications précédentes, dans lequel la sonde oligonucléotidique est choisie dans le groupe consistant en une sonde ayant une séquence de bases 5'-3' ATC TGA CCG TCC CAG GTT ; une sonde ayant une séquence de bases 5'-3' ATCTG ACCGT CCCAG GTT ; et une sonde ayant une séquence de bases 5'-3' AGCTT TCATC CAAAG ATA.

5. Procédé selon l'une quelconque des revendication précédentes, dans lequel, dans l'étape (2), ledit échantillon est mis en contact avec au moins une première sonde et une deuxième sonde, la première sonde étant apte à s'hybrider d'une manière spécifique avec au moins une partie des acides nucléiques ribosomiques appartenant tous à *Legionella genus,* et la deuxième sonde étant apte à s'hybrider d'une manière spécifique avec au moins une partie des acides nucléiques ribosomiques appartenant tous à l'espèce *Legionella pneumophila*.

6. Procédé selon la revendication 5, dans lequel la première sonde est choisie dans le groupe consistant en une sonde ayant une séquence de bases 5'-3' CTGGT GTTCC TTCCG ATC ; une sonde ayant une séquence de bases 5'-3' TCGGA CGCAG GCTAA TCT ; une séquence de bases 5'-3' CCTCC TCCCC ACTGA AAGT ; une sonde ayant une séquence de bases 5'-3' CACTG TATGT CAAGG GTAGG ; et une sonde ayant une séquence de bases 5'-3' AAGGC ATATT CCTAC GCG.

7. Procédé selon l'une quelconque des revendications précédente, dans lequel l'étape (3) est mise en oeuvre automatiquement par utilisation d'un microscope à épifluorescence.

8. Procédé selon l'une quelconque des revendications précédentes, dans lequel l'échantillon dérive de l'un quelconque du groupe choisi parmi les eaux de refroidissement industrielles, les eaux potables et les eaux naturelles.

9. Trousse pour détecter et dénombrer plus rapidement les organismes viables de l'espèce *Legionella pneumophila* dans un échantillon que l'on suspecte de contenir lesdits micro-organismes, comprenant :
(1) une ressource nutritive pour les cellules, qui contient un supplément de croissance, qui comprend un antioxydant, de préférence l'acide pyruvique (ou un sel de ce dernier) ;
(2) un inhibiteur de prolifération cellulaire ;
(3) des sondes oligonucléotidiques marquées par fluorescence, aptes à s'hybrider d'une manière spécifique avec au moins une partie des acides nucléiques ribosomiques desdits micro-organismes, comprenant une première sonde et une deuxième sonde, la première sonde étant apte à s'hybrider d'une manière spécifique avec au moins une partie des acides nucléiques ribosomiques desdits micro-organismes, et la deuxième sonde étant apte à s'hybrider d'une manière spécifique avec au moins une partie différente des acides nucléiques ribosomiques micro-organismes ;
(4) un moyen pour détecter et quantifier le signal fluorescent, l'inhibiteur de prolifération cellulaire étant choisi dans le groupe consistant en la ciprofloxacine et la céphalexine.
